# FASCICULE DE BREVET EUROPEEN

(11) **EP 3 673 926 B1**
(45) Date de publication et mention de la délivrance du brevet: **06.07.2022**
(21) Numéro de dépôt: 20157466.2
(22) Date de dépôt: 20.06.2016
(51) Int. Cl.: A61L 27/42, A61K 35/618, A61L 24/02, A61L 27/36, A61L 27/44, A61L 27/58, A61P 17/02, A61P 17/06

(54) **MATERIAU SEMI-SYNTHETIQUE PULVERULENT, OBTENU PAR MODIFICATION DE LA COMPOSITION D'UN BIOMATERIAU NATUREL MARIN, SON PROCEDE DE FABRICATION, SES APPLICATIONS**
HALBSYNTHETISCHES PULVERFÖRMIGES MATERIAL, DAS DURCH VERÄNDERUNG DER ZUSAMMENSETZUNG EINES MARINEN NATÜRLICHEN BIOMATERIALS ERHALTEN WIRD, SEIN HERSTELLUNGSVERFAHREN UND SEINE ANWENDUNGEN
SEMI-SYNTHETIC POWDERY MATERIAL OBTAINED BY MODIFYING THE COMPOSITION OF A NATURAL MARINE BIOMATERIAL, METHOD FOR MANUFACTURING SAME, USES THEREOF

(30) Priorité: 23.06.2015 FR 1555782
(43) Date de publication de la demande: 01.07.2020
(62) Demande divisionnaire de: 16741662.7
(73) Titulaire: MBP (Mauritius) Ltd, Port Louis (MU)
(72) Inventeur: CAMPRASSE, Georges, 72230 ARNAGE (FR); CAMPRASSE, Serge, 72100 Le Mans (FR)
(74) Mandataire: Plasseraud IP

(56) Documents cités:
- WO-A1-90/14111

## Description

### Domaine de l'invention

La présente invention concerne une utilisation des bio-polymères insolubles et solubles extraits de la fraction organique de la couche aragonitique interne et/ou de la couche calcitique externe de la coquille de mollusques bivalves choisis parmi les Pinctadines en général et notamment *Pinctada maxima*, *margaritifera*, et les Tridacnes : *Tridacna gigas*, *maxima, derasa, tevaroa*, *squamosa*, *crocea, Hippopus hippopus*, *Hippopus porcelanus.*

### Contexte de l'invention

WO9014111 décrit un produit de remplacement de structures osseuses réalisé à partir de couche nacrée du test de mollusques aquatiques.

Généralement les matériaux utilisés pour le comblement des pertes de substance osseuse d'origine traumatique, tumorale, dystrophique ou dégénérative, sont des ciments phosphocalciques, des bio-copolymères, des matériaux d'origine animale ou humaine.

Concernant le scellement des prothèses, seul le poly(méthacrylate de méthyle) (PMMA) est utilisé, associé éventuellement à des antibiotiques, un initiateur, un activateur, un opacifiant ou un colorant. Les endoprothèses sont généralement scellées par des ciments au PMMA, dont les inconvénients sont parfaitement connus, en particulier la réaction exothermique produite lors de la polymérisation du ciment, la nécrose cellulaire osseuse qui en résulte, la rétraction du ciment dans le temps et son vieillissement, qui provoquent la mobilité de la prothèse et la nécessité de sa reprise dans les 10 à 15 ans postopératoires, dans la majorité des cas.

Tous ces matériaux sont biocompatibles, certains d'entre eux, tels que les ciments phosphocalciques, revendiquent des propriétés ostéoconductrices ; peu sont bioactifs, la majorité étant inerte.

Les ciments injectables sont constitués d'une phase minérale et d'une phase liquide qui peut être l'acide phosphorique, une solution aqueuse ou de gel de HPMC, d'eau stoechiométrique de 0,1 mole, d'acide sulfurique, d'acide citrique.

Les biomatériaux, synthétiques ou d'origine bovine, utilisés comme substituts osseux, sont essentiellement dotés de propriétés ostéoconductrices et ne sont généralement pas totalement bio-résorbables.

Pour certains d'entre eux, notamment les polymères, on constate la libération de produits de dégradation qui peuvent avoir à long terme des effets nocifs sur les tissus environnants ou systémiques morbides. Cette bio-résorption est patient-dépendante.

Par ailleurs, la quasi-totalité des substituts osseux ne sont pas bioactifs ; ce qui oblige à les associer à du collagène d'origine animale, ou à d'autres substances qui, pour être bio-résorbées, induisent une réaction inflammatoire majeure de l'hôte receveur, plus importante et différente de la réaction physiologique.

L'inconvénient majeur des substituts osseux sous forme pulvérulente ou de granules, réside dans le fait que lors de leur mise en œuvre, que ce soit avec le sang autologue, le sérum physiologique ou tout autre vecteur liquide, ils ne forment pas de « coagulum » ayant des propriétés adhésives et plastiques favorisant leur cohésion et leur maintien sur et dans le site.

On sait que l'os humain est constitué de 43% de composants inorganiques, de 32% de composants organiques et de 25% d'eau. La composante organique est constituée à 90% de protéines collagéniques -dont 97% de collagène de type I, de type III, IV et V-, ainsi que 10% de protéines non collagéniques représentées par l'ostéocalcine, l'ostéonectine, l'ostéopontine, la sialo-protéine osseuse, les protéoglycanes, la fibronectine, les facteurs de croissance et les protéines morphogéniques. Ce sont ces protéines non collagéniques qui jouent un rôle essentiel dans les processus de l'ostéogénèse et de la réparation des tissus lésés.

La fraction inorganique est constituée en grande partie d'hydroxyapatite sous forme de cristaux de phosphate de calcium; cette fraction contient également d'autres minéraux tels que le sodium, le potassium, le cuivre, le zinc, le strontium, le fluor, l'aluminium, le silicium en très faibles quantités. Tous ces éléments jouent un rôle important dans le métabolisme cellulaire ainsi que dans la cicatrisation et la régénération osseuse.

L'étude de l'architecture et de la composition de la coquille des mollusques bivalves, tels que les Pinctadines en général et notamment *Pinctada maxima*, *margaritifera*, et les Tridacnes, notamment *Tridacna gigas, maxima, derasa, tevaroa, squamosa*, *crocea, Hippopus hippopus*, *Hippopus porcelanus*, a montré qu'elle comprend une couche interne nacrée, composée de 3 à 5% d'une fraction organique, elle-même constituée de protéines collagéniques et non collagéniques, essentiellement des biopolymères insolubles et solubles. La couche interne nacrée contient également une fraction inorganique représentant 95 à 97%, constituée essentiellement de carbonate de calcium, de minéraux et d'ions métalliques, ainsi que de 3% d'eau. Cette étude de l'architecture de la coquille des mollusques concernés par l'invention montre aussi qu'elle est constituée d'une couche calcitique externe, structuralement différente de la couche aragonitique interne, mais contenant également une fraction organique composée de bio-polymères insolubles et solubles.

De nombreuses publications ont mis en évidence les propriétés ostéo-inductrices et ostéo-conductrices du biomatériau naturel issu de la couche aragonitique des mollusques marins cités plus haut.

Ces propriétés découlent de la présence de bio-polymères contenus dans la fraction organique, dans laquelle on a identifié des protéines structurales analogues à celles qui contribuent à l'architecture d'organes comme les dents, les os, la peau, les muscles, les muqueuses, etc ... Sont également présentes des protéines fonctionnelles semblables à celles qui interviennent dans les processus métaboliques, biochimiques (enzymologie, immunologie, récepteurs membranaires, molécules signal, etc...). Parmi ces protéines structurales, les collagènes sont particulièrement représentés : c'est ainsi que l'on a identifié des collagènes de type I, II, III et apparentés.

En dehors des acides aminés libres, on a mis en évidence la présence de protéoglycanes (glucides liés à des petits peptides), de glycoprotéines (association de collagène et de glucides) parmi lesquelles des glycoprotéines de faible poids moléculaire, généralement considérées comme des facteurs de croissance apparentés à la BMP, à TNF β, TGF β, PGF, etc ...

On connait par ailleurs le rôle fondamental de certaines molécules non collagéniques dans le processus de cicatrisation physiologique et dans la régénération cellulaire et tissulaire.

In vitro et in vivo, on a mis en évidence les propriétés de cicatrisation, de régénération, d'angiogénèse et d'ostéo-induction du complexe organo-minéral de la couche interne de la coquille des mollusques cités plus haut, propriétés liées à la présence de ces différents collagènes et facteurs de croissance.

Si l'on compare la composition physico-chimique du tissu osseux et celle de l'aragonite des coquilles des mollusques considérés, on note une forte similitude des composants organiques présents à un pourcentage de 32% dans le tissu osseux et de 3 à 5 % dans l'aragonite. Les phases minérales, 43% pour l'os, essentiellement du phosphate de calcium, représentent dans l'aragonite 95 à 97% sous forme de carbonate de calcium ; les proportions des autres minéraux étant très proches.

Compte tenu du rôle des bio-polymères contenus dans la fraction organique du biomatériau naturel marin, les inventeurs ont trouvé judicieux d'en modifier la composition en augmentant la proportion de ces bio-polymères dans la composition d'un nouveau biomatériau hybride semi-synthétique.

On sait que la fraction organique des couches aragonitique interne et calcitique externe des coquilles des mollusques concernés, contient des molécules solubles, diffusibles, ayant des propriétés ostéogènes intervenant dans la minéralisation et la croissance des tissus calcifiés. On a également mis en évidence la présence de protéines insolubles, de structure, dans les enveloppes péri-cristallines et inter-lamellaires de l'aragonite.

Par ailleurs, les molécules contenues dans la fraction organique de la couche calcitique externe de la coquille sont semblables à celles contenues dans la couche aragonitique interne de la coquille des mollusques concernés par l'invention.

C'est la raison pour laquelle, il est apparu judicieux d'extraire et de concentrer, non seulement les molécules organiques étroitement liées aux biocristaux et aux lamelles intercristallines dont est constituée l'aragonite des tests nacrés, mais également celles contenues dans la couche calcitique externe des coquilles des mollusques concernés.

L'extraction des bio-polymères des fractions organiques du biomatériau a pour objet de mettre à disposition des molécules solubles et insolubles. L'objectif est de pouvoir augmenter, par supplémentation en bio-polymères insolubles et solubles extraits, le rapport structural organique-inorganique, afin d'optimiser les propriétés de régénération cellulaire et tissulaire, de cicatrisation, d'ostéo-induction, d'angiogénèse du biomatériau ainsi obtenu.

C'est ainsi que les présents inventeurs ont trouvé qu'il est possible, à partir de la coquille d'un mollusque choisi parmi *Tridacnae maxima*, *Tridacnae gigas*, *Tridacnae derasa, Tridacnae tevaroa*, *Tridacnae squamosa*, *Tridacnae crocea, Hippopus hippopus*, *Hippopus porcelanus*, *Pinctada maxima*, *Pinctada margaritifera*, et autres Pinctadines, d'obtenir un matériau satisfaisant ces exigences en l'adjuvantant à la fois en biopolymères solubles et insolubles et en carbonate de calcium transformé par carbonatation.

Ainsi modifié, le nouveau matériau semi-synthétique pulvérulent bio-résorbable, est destiné à la fabrication par exemple, de substituts osseux, de ciments injectables ou de ciments de scellement d'endoprothèses, ou encore à l'élaboration de dispositifs d'ostéosynthèse et d'implants moulés, bio-résorbables.

Ainsi, l'invention a pour objet, selon un premier aspect, une utilisation des bio-polymères insolubles et solubles extraits de la fraction organique de la couche aragonitique interne et/ou de la couche calcitique externe de la coquille des mollusques bivalves choisis dans le groupe comprenant des Pinctadines, notamment *Pinctada maxima*, *margaritifera*, et des Tridacnes, notamment *Tridacna gigas*, *maxima*, *derasa, tevaroa*, *squamosa*, *crocea, Hippopus hippopus*, *Hippopus porcelanus* comme adjuvants de composition pulvérulente comprenant des sels de calcium, des polymères naturels ou synthétiques, du collagène, des trames minérales de tissus osseux d'origine animale ou humaine,
caractérisée en ce que la composition pulvérulente est un matériau semi-synthétique pulvérulent issu d'un biomatériau naturel marin additionné de carbonate de calcium transformé par carbonatation, ledit biomatériau naturel marin étant la couche aragonitique interne de la coquille de mollusques bivalves choisis dans le groupe comprenant des Pinctadines, notamment *Pinctada maxima*, *margaritifera*, et des Tridacnes, notamment *Tridacna gigas*, *maxima, derasa, tevaroa*, *squamosa*, *crocea, Hippopus hippopus*, *Hippopus porcelanus.*

### Description détaillée de l'invention :

Selon un premier aspect, l'invention porte sur une utilisation des bio-polymères insolubles et solubles extraits de la fraction organique de la couche aragonitique interne et/ou de la couche calcitique externe de la coquille des mollusques bivalves choisis dans le groupe comprenant des Pinctadines, notamment *Pinctada maxima*, *margaritifera*, et des Tridacnes, notamment *Tridacna gigas*, *maxima*, *derasa, tevaroa*, *squamosa*, *crocea, Hippopus hippopus*, *Hippopus porcelanus* comme adjuvants de composition pulvérulente comprenant des sels de calcium, des polymères naturels ou synthétiques, du collagène, des trames minérales de tissus osseux d'origine animale ou humaine,
caractérisée en ce que la composition pulvérulente est un matériau semi-synthétique pulvérulent issu d'un biomatériau naturel marin additionné de carbonate de calcium transformé par carbonatation, ledit biomatériau naturel marin étant la couche aragonitique interne de la coquille de mollusques bivalves choisis dans le groupe comprenant des Pinctadines, notamment *Pinctada maxima*, *margaritifera*, et des Tridacnes, notamment *Tridacna gigas*, *maxima, derasa, tevaroa*, *squamosa*, *crocea, Hippopus hippopus*, *Hippopus porcelanus.*

Le matériau semi-synthétique pulvérulent selon l'invention est bio-résorbabale.

Selon un mode de réalisation, la granulométrie est de 5 nm à 100 µm, de préférence de 20 nm à 50 µm, plus préférentiellement encore de 50 nm à 20 µm.

Les biopolymères solubles et insolubles sont extraits de la couche aragonitique interne et/ou de la couche calcitique externe de la coquille des mollusques bivalves choisis dans le groupe comprenant des Pinctadines, notamment *Pinctada maxima*, *margaritifera*, et des Tridacnes, notamment *Tridacna gigas, maxima*, *derasa, tevaroa*, *squamosa*, *crocea, Hippopus hippopus*, *Hippopus porcelanus.*

Une méthode d'extraction de ces polymères est décrite ci-après.

Le carbonate de calcium transformé par carbonatation mis en œuvre dans l'invention, provient d'un carbonate de calcium naturel terrestre, marin ou précipité, ou de la fraction inorganique de la couche aragonitique après extraction des bio-polymères insolubles et solubles, qui a été transformé par carbonatation. On sait que le carbonate de calcium, cristallisé dans le système orthorhombique ou rhomboédrique, lorsqu'il est soumis à un traitement thermique entre 800 et 1100° C, acquiert par thermolyse et oxydation, des propriétés nouvelles qui se traduisent par un pouvoir adhésif important et une plasticité permettant un modelage aisé. Ce phénomène est la carbonatation, selon la réaction suivante :

CaCO₃ + traitement thermique → Ca(OH)₂ + CO₂ → CaCO₃ + H₂O

Lors de cette réaction, au cours de laquelle se produit l'élévation de la température et son maintien pendant une durée de 20 à 40 mn, le carbonate de calcium se transforme chimiquement en devenant de la chaux, puis sous l'action du CO₂ et de l'humidité ambiante, devient du carbonate de calcium amorphe. Cette transformation chimique s'étale sur plusieurs jours en fonction de l'hygrométrie ambiante.

Ainsi, le matériau semi-synthétique pulvérulent comprend une poudre issue d'un matériau naturel marin dont la fraction organique est supplémentée en bio-polymères insolubles et solubles extraits, et la fraction minérale en carbonate de calcium d'origine marine, sédimentaire ou madréporaire, ou d'origine terrestre sédimentaire ou précipité, transformé par un procédé de carbonatation.

Selon un mode de réalisation particulier, le matériau semi-synthétique pulvérulent comprend de l'aragonite sous forme pulvérulente d'une granulométrie 5 nm à 100 µm, de préférence de 20 nm à 50 µm, plus préférentiellement encore de 50 nm à 20 µm, des biopolymères insolubles et solubles extraits, et le carbonate de calcium transformé par carbonatation.

Par l'ajout des biopolymères insolubles et solubles extraits, la part de la fraction organique du matériau initial est augmentée dans une fourchette comprise entre 1% et 10%, de préférence en respectant les proportions entre bio-polymères insolubles et biopolymères solubles existant dans le matériau de départ. Par l'ajout de carbonate de calcium transformé par carbonatation, la part de la fraction minérale du matériau initial est augmentée dans une fourchette comprise entre 1% et 10%, en fonction des caractéristiques physico-chimiques souhaitées.

Selon un mode de réalisation particulier, le matériau semi-synthétique comprend : pour 100g d'aragonite sous forme pulvérulente d'une granulométrie 5 nm à 100 µm, de préférence de 20 nm à 50 µm, plus préférentiellement encore de 50 nm à 20 µm ;
de 1 g à 50 g, de préférence de 5 g à 25 g, plus préférentiellement encore de 10 g à 15 g de bio-polymères insolubles et solubles extraits ; et
de 0,5 g à 50 g, de préférence de 1 g à 25 g, plus préférentiellement encore de 2 g à 10 g de carbonate de calcium transformé par carbonatation.

Lors de l'extraction des bio-polymères, les inventeurs ont mis en évidence que dans la couche aragonitique interne et la couche calcitique externe des mollusques utilisés dans la mise en œuvre de l'invention, la part des bio-polymères insolubles représente de 2,6% à 4,3% et celle des bio-polymères solubles de 0,4% à 0,7% du poids total. L'ajout de biopolymères dans le matériau décrit, se fait de telle sorte que le ratio biopolymères solubles / biopolymères insolubles soit similaire au ratio du produit naturel d'origine.

Il est décrit un procédé de préparation d'un matériau semi-synthétique pulvérulent, tel que décrit précédemment.

Selon le procédé décrit les éléments constitutifs sont préparés séparément puis mélangés de façon à obtenir le matériau décrit. Ainsi, on prépare le matériau pulvérulent issu d'un biomatériau naturel marin, les bio-polymères insolubles et solubles extraits d'un biomatériau naturel marin et le carbonate de calcium transformé par carbonatation.

Plus particulièrement, le procédé de préparation comprend le mélange d'un biomatériau naturel broyé, de polymères insolubles et solubles extraits de la couche aragonitique interne et/ou de la couche calcitique externe de la coquille des mollusques bivalves choisis dans le groupe comprenant des Pinctadines, notamment *Pinctada maxima, margaritifera*, et des Tridacnes, notamment *Tridacna gigas, maxima, derasa, tevaroa, squamosa*, *crocea, Hippopus hippopus*, *Hippopus porcelanus* et de carbonate de calcium transformé par carbonatation.

Dans un mode de réalisation particulier, le biomatériau naturel broyé est la couche aragonitique interne de la coquille des mollusques. Le broyage est réalisé de façon à obtenir une granulométrie moyenne de 20nm à 50µm. Les grains obtenus peuvent être sphéronisées pour améliorer la coulabilité et la compressibilité de la poudre.

Dans le procédé décrit, les bio-polymères insolubles et solubles sont extraits respectivement, par super-centrifugation, et par ultrafiltration tangentielle couplée à une osmose inverse après hydrolyse. Avant l'extraction, la couche aragonitique interne et/ou la couche calcitique externe de la coquille des mollusques peuvent(peut) être réticulée(s). Pour faciliter l'extraction,
la couche aragonitique interne et/ou la couche calcitique externe de la coquille des mollusques sont(est) broyée(s) et tamisée(s) à une granulométrie comprise entre 250 µm et 50 µm.

Ces différentes étapes sont décrites successivement dans ce qui suit.

Le biomatériau naturel marin utilisé comme matière première est choisi dans le groupe comprenant des Pinctadines, notamment *Pinctada maxima*, *margaritifera*, et des Tridacnes, notamment *Tridacna gigas*, *maxima*, *derasa, tevaroa*, *squamosa*, *crocea, Hippopus hippopus*, *Hippopus porcelanus.*

Chacun des composants peut être issu du même biomatériau marin ou de biomatériaux marins différents.

Les coquilles choisies sont nettoyées, décontaminées, éventuellement réticulées, la couche calcitique est séparée de la couche interne. La couche interne est broyée. Une partie de la couche interne broyée constitue le composant de base du matériau décrit. Les biopolymères solubles et insolubles sont extraits de la couche calcitique et/ou de la couche interne. Le carbonate de calcium qui peut provenir de la partie minérale récupérée après extraction des biopolymères est transformé par carbonatation. Les biopolymères ainsi extraits et le carbonate de calcium transformé par carbonatation sont ajoutés au composant de base préalablement obtenu.

Un mode de réalisation spécifique du procédé est décrit de façon détaillée dans ce qui suit. Bien entendu, l'homme du métier saura adapter les conditions de ce procédé aux biomatériaux spécifiques de départ et aux utilisations finales souhaitées.

### I. PREPARATION DES COMPOSANTS :

Après élimination de l'épiobonte par grattage, les coquilles provenant du biomatériau marin choisi subissent les traitements suivants :

### I.1) Décontamination des coquilles :

Les coquilles sont décontaminées par immersion dans un bain d'eau du réseau additionné d'une solution d'hypochlorite à 2% de chlore actif.

### I.2) Traitement aux ultra-sons des coquilles :

Les coquilles sont ensuite rincées et traitées dans un bac à ultrasons rempli d'eau du réseau microbiologiquement contrôlée, par exemple à une température de 55°C à laquelle on ajoute une solution nettoyante et désinfectante à une dilution de 1 part de solution pour 127 parts d'eau. La durée du traitement étant de l'ordre de 30 mn à une fréquence d'environ 40 kHz.

### I.3) Rinçage et séchage des coquilles :

Les coquilles sont ensuite rincées par exemple pendant 20 mn dans un bain d'eau déminéralisée à une température de 90°C, additionnée de Calbénium^{®} à une dilution de 2%, pendant 30 mn. Elles sont ensuite séchées.

### I.4) Réticulation des coquilles :

Selon un autre mode de réalisation, afin de conférer au biomatériau d'origine naturelle des propriétés biologiques accrues, notamment en vue de l'optimisation du métabolisme cellulaire et du renforcement des propriétés anti-radicalaires, les coquilles peuvent être réticulées de la manière suivante :

Dans un récipient translucide en verre ou en plastique de contenance variable, on prépare un mélange d'eau du réseau additionnée de 10% de riboflavine ; l'ensemble est maintenu à une température supérieure à 20°C, l'agitation du mélange générant un flot perpendiculaire aux radiations UVA.

Les coquilles y sont placées verticalement et soumises sur leurs deux faces à l'irradiation de lampes UVA d'une longueur d'ondes de 365 nanomètres seconde, sous une intensité de 2300 micro-joules par centimètre carré pendant 180 mn. L'ensemble étant maintenu sous vide pendant toute la durée du traitement.

Les coquilles sont ensuite rincées et séchées par courant d'air chaud à 40°C.

On peut également utiliser le procédé décrit dans la demande de brevet FR 14 50204 déposée le 10 janvier 2014.

### I.5) Elimination de la couche calcitique externe:

La couche calcitique externe des coquilles est éliminée par meulage, avec une meule à grains fins.

Le produit est réservé et constitue le « Lot d'extraction des bio-polymères de la couche calcitique externe ».

### I.6) Congélation des tests nacrés exposés après meulage :

Les tests nacrés sont congelés à une température de -18°C pendant 120mn.

### I.7) Concassage des tests nacrés et récupération des lots :

On procède ensuite au concassage des tests nacrés par exemple dans un concasseur à mâchoires en carbure de tungstène, sous aspiration, de manière à récupérer les particules en suspension, contenant également des nano-grains.

L'opération de concassage est renouvelée au moins 3 fois et on réserve après tamisage, 2 lots :
- Le premier d'une granulométrie aléatoire de 20 microns à 50 nanomètres constituera la portion mixte aragonitique du produit décrit dénommée ci-après « lot mixte aragonitique ». On entend par lot mixte aragonitique la forme pulvérulente obtenue après broyage comprenant les deux composants organique et inorganique.
- Le second lot d'une granulométrie de 250 à 50 microns est réservé à l'extraction des biopolymères insolubles et solubles. Il sera dénommé « lot d'extraction des bio-polymères de la couche aragonitique interne ».

L'utilisation d'un granulomètre laser déterminera la taille et l'éventail des grains des poudres obtenues.

### I.8) Sphérification du lot mixte aragonitique :

Le lot mixte aragonitique est soumis à un traitement mécanique destiné à uniformiser les grains par sphérification, le but étant d'arrondir les angles et les arêtes des grains par frottement.

Ce traitement a pour effet de favoriser la coulabilité, la comprimabilité, de la poudre obtenue et ainsi de favoriser la densification et les liaisons inter-particulaires lors de la mise en œuvre du matériau décrit, notamment comme substituts osseux, ciments de scellement, ciments injectables, dispositifs d'ostéosynthèse et implants moulés bio-résorbables.

Pour cette étape de sphérification, on peut procéder de la façon suivante : on place dans un récipient cylindrique en verre ou en zirconium par exemple, à axe de rotation horizontal, comportant des pales en verre d'une largeur variable, un mélange à parts égales de matériau pulvérulent du lot mixte aragonitique et de copeaux de quelques mm² de bois dur, par exemple du chêne, stérilisés à l'autoclave.

Le récipient est mis en rotation pendant une durée et à une vitesse variables, dépendant de la taille du récipient et de la quantité de produit à traiter.

A l'issue du traitement de sphérification, l'ensemble du mélange, lot mixte aragonitique et copeaux, est récupéré dans un récipient inerte rempli d'une quantité adéquate d'eau que l'on agite de manière constante pendant 15 mn environ. Après repos, les copeaux de bois flottant en surface sont éliminés par aspiration.

La solution est ensuite filtrée sur un filtre en nylon aux mailles d'un diamètre de 20 microns, puis le culot est séché au Rotavapor^{®} à 40°C et conditionné.

Selon un autre mode de réalisation, le lot mixte aragonitique peut également être additionné à parts égales de chlorure de sodium sous forme de grains de diamètres aléatoires allant de 1 à 3 mm. Après traitement, on élimine le chlorure de sodium par dissolution à l'eau chaude à 90° C et filtration sur filtre de nylon, suivie d'un lavage à l'eau chaude à 90° C et séchage par flux d'air chaud à 40° C.

### II. EXTRACTION DES BIO-POLYMERES

### II.1 Extraction des bio-polymères insolubles :

Selon l'invention, une quantité adéquate de poudre du lot d'extraction des biopolymères de la couche aragonitique interne obtenue à l'étape I.5), est mélangée avec une quantité suffisante d'eau déminéralisée, pour être injectée dans un réacteur à hydrolyse, dans lequel est ajoutée une quantité déterminée d'acide citrique à 25% ; le tout étant refroidi à une température oscillant entre 4 et 5°C sous agitation constante. Les inventeurs ont privilégié l'utilisation de l'acide citrique en raison de ses propriétés d'abaisseur de pH et de tension superficielle.

Le pH, contrôlé par le pH mètre est maintenu au dessus de 4,5 par adjonction de soude 2,5 N afin d'éviter d'altérer les bio-polymères ; il est ensuite ramené à 7 en fin d'étape par adjonction de 0,1 litre de soude 5N pour 100 litres d'hydrolysat.

Une fois obtenue la dissolution complète de la poudre, l'hydrolysat est transféré dans une cuve de stockage, toujours sous agitation constante, puis transféré dans un séparateur centrifuge où il est soumis à une force de 18 à 20 000G dans le cyclone.

L'opération est répétée si nécessaire après contrôle de la solution par le turbidimètre et correction à l'acide citrique si besoin, la température étant maintenue entre 4 et 5°C.

Selon les résultats fournis par le turbidimètre, l'hydrolysat peut à nouveau subir une super centrifugation.

A chaque cycle de super centrifugation, le culot des bio-polymères insolubles récolté est lavé et réservé. Les eaux de lavage des culots sont traitées à l'acide oxalique pour vérifier la présence ou non de calcium.

A l'issue de la dernière super centrifugation, on obtient donc un culot contenant tous les bio-polymères insolubles, sous la forme d'un gâteau brunâtre humide, qui est séché par lyophilisation, ou zéodratation, avec en fin de traitement des sphérules grises de 2 à 3 mm de diamètre, résultant de l'enroulement des protéines sous l'action de la force centrifuge.

Les bio-polymères insolubles extraits sont broyés par exemple dans un broyeur planétaire jusqu'à l'obtention d'une poudre d'une granulométrie aléatoire de 5 microns à 100 nanomètres récupérée après tamisage.

### II.2 Extraction des bio-polymères solubles :

Le perméat et les eaux de lavage sont acheminés pour être dessalés dans un dispositif d'ultra filtration tangentielle, par exemple avec des cassettes présentant un seuil de coupure de 1kD.

On ajoute au perméat une quantité suffisante d'acide sulfurique à 2,0 mol/L , afin de provoquer la précipitation des sels de sulfate de calcium.

La solution est filtrée, le perméat est concentré au Rotavapor^{®} sous vide à une température d'ébullition de 33°C afin d'éliminer l'acide citrique sous forme de cristaux.

Le distillat contenant les protéines de faible poids moléculaire ainsi que les ions mono et multivalents, est allongé.

Le seuil de coupure des cassettes ne retenant pas la totalité des protéines et notamment celles de très faible poids moléculaire, le distillat est soumis à une osmose inverse.

Le distillat est transféré pour subir un traitement de séparation en phase liquide par perméation à travers des membranes semi-sélectives par exemple de diamètre des pores de 0,0001 micron, sous l'effet d'un gradient de pression de 40 à 80 bars.

Le distillat est passé afin de retenir tous les ions mono- et multivalents tels que le fer, le magnésium, le zinc, etc ...

Le rétentat récupéré sur les membranes d'osmose inverse est recueilli et allongé avec de l'eau apyrogène, puis concentré par exemple par Rotavapor^{®}, sous vide, à une température de 40°C, puis lyophilisé par zéodratation ou cryodessication.

On obtient une poudre très fine de couleur blanc grisâtre qui est réservée, puis broyée par exemple dans un broyeur planétaire afin d'obtenir après tamisage une poudre d'une granulométrie aléatoire allant de 5 microns à 100 nanomètres.

On vérifie la présence ou l'absence de protéines dans le perméat en prélevant une aliquote de solution qui est traitée par la méthode colorimétrique de Bradford.

### II.3 Extraction des bio-polymères du lot d'extraction des bio-polymères de la couche calcitique externe

Selon un autre mode de réalisation, l'extraction des bio-polymères de la couche calcitique externe est effectuée d'une manière identique à celle des bio-polymères de la couche aragonitique interne.

### III. CARBONATATION DU CARBONATE DE CALCIUM

On sait que le carbonate de calcium, cristallisé dans le système orthorhombique ou rhomboédrique, lorsqu'il est soumis à un traitement thermique entre 800 et 1100° C, acquiert par thermolyse et oxydation, des propriétés nouvelles qui se traduisent par un pouvoir adhésif important et une plasticité permettant un modelage aisé. Ce phénomène est la carbonatation, selon la réaction suivante :

CaCO₃ + traitement thermique → Ca(OH)₂ + CO₂ → CaCO₃ + H₂O

Lors de cette réaction, au cours de laquelle se produit l'élévation de la température et son maintien pendant une durée de 20 à 40 mn, le carbonate de calcium se transforme chimiquement en devenant de la chaux, puis sous l'action du CO₂ et de l'humidité ambiante, devient du carbonate de calcium amorphe. Cette transformation chimique s'étale sur plusieurs jours en fonction de l'hygrométrie ambiante.

Selon d'autres modes de réalisation, tous les sels de calcium, autres que le carbonate de calcium, peuvent, par des réactions chimiques de précipitation, donner naissance à du carbonate de calcium pouvant être transformé par carbonatation. C'est ainsi que l'on peut, par exemple, obtenir du carbonate de calcium carbonaté à partir de l'hydroxyde de calcium, de l'acétate de calcium, de l'oxalate de calcium, du sulfate de calcium, du citrate de calcium ; il appartiendra à l'homme de métier de mettre en oeuvre les procédés chimiques connus propres à ces précipitations.

Le carbonate de calcium peut également provenir de la coquille interne aragonitique des mollusques bivalves tels que les Pinctadines en général et notamment Pinctada : maxima, margaritifera, et les Tridacna : gigas, maxima, derasa, tevaroa, squamosa, crocea, Hippopus hippopus, Hippopus porcelanus, après extraction des biopolymères. Il peut également être d'origine madréporaire.

### IV. FORMULATION D'UN MELANGE A PARTIR DU LOT MIXTE ARAGONITIQUE, DES BIO-POLYMERES INSOLUBLES ET SOLUBLES EXTRAITS, ET DU CARBONATE DE CALCIUM TRANSFORME PAR CARBONATATION

Une quantité des bio-polymères insolubles et solubles, extraits à partir des deux lots aragonitique interne et calcitique externe, déterminée selon la part de fraction organique souhaitée, et une quantité déterminée de carbonate de calcium transformé par carbonatation, sont mélangées à une quantité définie du lot mixte aragonitique pour constituer une formulation du produit décrit.

Le mélange est effectué par exemple dans un mélangeur à couteaux jusqu'à obtention d'une poudre homogène qui est ensuite conditionnée.

Selon un autre aspect, l'invention porte sur un matériau semi-synthétique pulvérulent, issu d'un biomatériau naturel marin, additionné de bio-polymères insolubles et solubles extraits de la fraction organique de la couche aragonitique interne et/ou de la couche calcitique externe de la coquille des mollusques bivalves choisis dans le groupe comprenant des Pinctadines, notamment *Pinctada maxima, margaritifera,* et des Tridacnes, notamment *Tridacna gigas, maxima, derasa, tevaroa, squamosa*, *crocea, Hippopus hippopus*, *Hippopus porcelanus* et de carbonate de calcium transformé par carbonatation pour son utilisation pour la cicatrisation et la régénération des pertes de substance ou pour le traitement de brûlures, d'escarres, d'ulcères, de lésions erythémateuses cutanées ou l'utilisation du matériau décrit dans la fabrication de dispositifs ou d'implants moulés.

Le matériau semi-synthétique pulvérulent décrit peut également être utilisé dans la fabrication de dispositifs ou d'implants moulés à bio-résorption contrôlée comprenant des fils de suture à bio-résorption échelonnée dans le temps.

Il peut aussi être utilisé pour la formulation de préparations pour substituts osseux à utilisation extemporanée, de substituts osseux à support collagénique poreux, de substituts osseux à trame minérale d'origine animale ou humaine, de dispositifs d'ostéosynthèse et d'implants moulés bio-résorbables, de dispositifs à bio-résorption contrôlée, de ciments de scellement d'endoprothèses, de ciments injectables pour chirurgie mini-invasive en vertébroplastie, cyphoplastie et la chirurgie tumorale osseuse.

Selon un autre mode de réalisation, le produit décrit, peut être associé à un support collagénique poreux tel que Spongia officinalis ayant subi un traitement mécanique et thermochimique destiné à la décontamination bactérienne et virale, à l'élimination des pigments éventuels, à la neutralisation d'une manifestation immunogène. On sait que Spongia officinalis est composée de Spongine, elle-même constituée de fibres d'une scléroprotéine carbonatée apparentée à un collagène. Cette protéine est peu soluble et joue un rôle de protection et de soutien de tous les tissus : le tissu conjonctif, les tendons, les tissus osseux, les fibres musculaires, la peau, les poils et les ongles. La Spongine est une protéine collagénique de structure et de stockage ; elle est inerte, insoluble dans l'eau, hydrophobe et ne se dénature pas facilement. Elle constitue un support poreux, propre à l'ostéo-conduction. Elle peut donc être utilisée en combinaison avec le matériau décrit pour la réalisation de substituts osseux.

Le matériau décrit peut être associé à des sels de calcium tels que le sulfate de calcium déshydraté ou hémi-hydraté, la calcite, l'hydroxyphosphate de calcium anhydre, le β-TCP, l'hydroxyde de calcium. Le matériau décrit peut être associé aux trames minérales de tissus osseux d'origine animale ou humaine.

Il peut être également associé à des polymères bio-résorbables tels que le collagène, l'acide hyaluronique, le chitosan, l'amidon, l'alginate ou encore à des polymères synthétiques résorbables tels que le polyglycolide, le poly (DL-Lactide-co-glycolide) le poly (L-lactide) ou à des polymères acryliques tels que le poly hydroxyéthyl, le méthyl méthacrylate, le poly méthacrylate de méthyl, ainsi qu'à des substances médicamenteuses sous forme pulvérulente, telles que des anti-inflammatoires non stéroïdiens, des antibiotiques, des antimitotiques ou toute autre substance à visée thérapeutique.

Compte tenu des inconvénients liés à l'utilisation des ciments de scellement au méthyl méthacrylate, les inventeurs proposent des ciments de scellement fabriqués avec le produit décrit, qui, naturellement radio-opaque, réalise une rétention primaire mécanique de l'endoprothèse en raison de ses propriétés adhésives, pour aboutir dans un second temps à une intégration tissulaire en raison de ses propriétés ostéo-mimétiques, ostéo-inductrices, ostéo-conductrices, bioactives, induites par la présence de molécules signal, initiatrices de la bio-minéralisation.

Ces molécules signal stimulent in situ les facteurs endogènes locaux de la bio-minéralisation pour aboutir à la formation d'os métaplasique.

Il est décrit l'utilisation de carbonate de calcium ayant subi une carbonatation tel que mis en œuvre dans le matériau décrit ou tel que préparé selon l'étape III du procédé décrit ci-dessus dans des compositions comprenant des sels de calcium, des polymères naturels ou synthétiques, du collagène, des trames minérales de tissus osseux d'origine animale ou humaine.

Il peut être également associé à des polymères bio-résorbables tels que le collagène, l'acide hyaluronique, le chitosan, l'amidon, l'alginate ou encore à des polymères synthétiques résorbables tels que le polyglycolide, le poly (DL-Lactide-co-glycolide) le poly (L-lactide) ou à des polymères acryliques tels que le poly hydroxyéthyl, le méthyl méthacrylate, le poly méthacrylate de méthyl, ainsi qu'à des substances médicamenteuses sous forme pulvérulente, telles que des anti-inflammatoires non stéroïdiens, des antibiotiques, des antimitotiques ou toute autre substance à visée thérapeutique.

On sait que les bio-polymères insolubles et solubles contenus dans la fraction organique des couches aragonitique et calcitique ont des propriétés de cicatrisation et de régénération, aussi bien des tissus durs comme l'os et le cartilage, que des tissus mous tels que la peau, les muscles et les muqueuses. On peut apparenter certains de ces biopolymères non collagéniques, notamment les glycoprotéines de faible poids moléculaire, à des facteurs de croissance comme les BMP, TNFβ, EGPF, TGFβ, IGF, FGF, etc... et également à des cytokines, médiateurs de l'inflammation.

L'invention porte sur l'utilisation des biopolymères solubles et insolubles mis en œuvre dans le matériau décrit ou tels qu'ils sont extraits par l'étape II du procédé décrit ci-dessus comme adjuvants de compositions pulvérulentes comprenant des sels de calcium, des polymères naturels ou synthétiques, du collagène, des trames minérales de tissus osseux d'origine animale ou humaine. Ils peuvent être également associés à des polymères bio-résorbables tels que le collagène, l'acide hyaluronique, le chitosan, l'amidon, l'alginate ou encore à des polymères synthétiques résorbables tels que le polyglycolide, le poly (DL-Lactide-co-glycolide) le poly (L-lactide) ou à des polymères acryliques tels que le poly hydroxyéthyl, le méthyl méthacrylate, le poly méthacrylate de méthyl, ainsi qu'à des substances médicamenteuses sous forme pulvérulente, telles que des anti-inflammatoires non stéroïdiens, des antibiotiques, des antimitotiques ou toute autre substance à visée thérapeutique. Ils sont associés au carbonate de calcium transformé par carbonatation.

L'invention va être décrite plus en détail à l'aide des exemples suivants donnés à titre d'illustration seulement et des dessins annexés, sur lesquels :

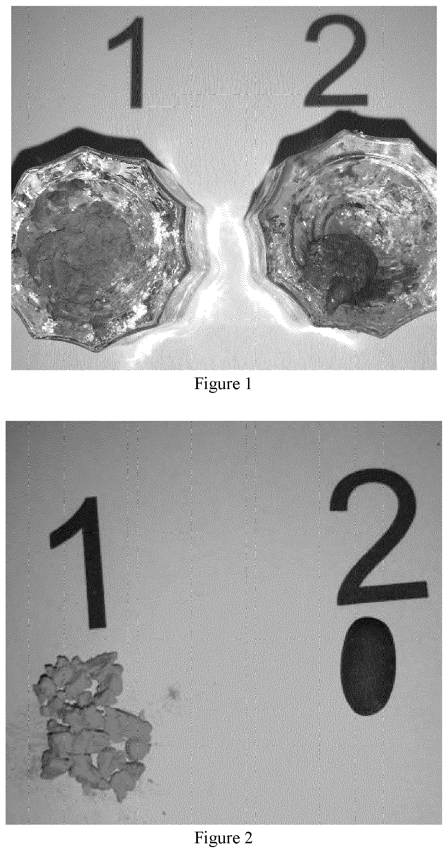

La Figure 1 et la Figure 2 sont des photographies de mélanges :
- de poudre de nacre et carbonate de calcium avec du sang total (N°1) et
- de poudre de nacre et carbonate de calcium ayant subi une carbonatation avec du sang total (n°2)
prises respectivement 2 mn puis 15 mn après ajout du sang total.

### EXEMPLES:

Afin de vérifier les propriétés pharmacologiques du produit décrit, les inventeurs ont procédé à la formulation de préparations à visée thérapeutique et leur utilisation dans des relevés d'observations cliniques.

### EXEMPLE 1 :

Le matériau semi-synthétique pulvérulent selon l'invention a été préparé de la façon suivante :

### I. PREPARATION DES COMPOSANTS :

Après élimination de l'épibionte par grattage, les coquilles subissent les traitements suivants :

### I.1) Décontamination des coquilles :

Les coquilles sont décontaminées par immersion dans un bain d'eau du réseau additionnée d'une solution d'hypochlorite à 2% de chlore actif.

### I.2) Traitement aux ultra-sons des coquilles :

Les coquilles sont ensuite rincées et traitées dans un bac à ultrasons rempli d'eau du réseau microbiologiquement contrôlée, à une température de 55°C à laquelle on ajoute une solution nettoyante et désinfectante à une dilution de 1 part de solution pour 127 parts d'eau. La durée du traitement étant de 30 mn à une fréquence de 40 kHz.

### I.3) Rinçage et séchage des coquilles :

Les coquilles sont ensuite rincées pendant 20 mn dans un bain d'eau déminéralisée à une température de 90°C, additionnée de Calbénium^{®} à une dilution de 2%, pendant 30 mn. Elles sont ensuite rincées et séchées.

### I.4) Elimination de la couche calcitique externe:

La couche calcitique externe des coquilles est éliminée par meulage, avec une meule à grains fins.

Le produit est réservé et constitue le « Lot d'extraction des bio-polymères de la couche calcitique externe ».

### I.5) Congélation des tests nacrés exposés après meulage :

Les tests nacrés obtenus à l'étape I.4) sont congelés à une température de -18°C pendant 120mn.

### I.6) Concassage des tests nacrés et récupération des lots :

On procède ensuite au concassage des tests nacrés dans un concasseur à mâchoires en carbure de tungstène, de marque ESSA^{®}, sous aspiration, de manière à récupérer les particules en suspension, contenant également des nano-grains.

L'opération de concassage est renouvelée au moins 3 fois et on réserve après tamisage, 2 lots :
- Le premier d'une granulométrie aléatoire de 20 microns à 50 nanomètres constituera la portion mixte aragonitique du produit décrit dénommée ci-après « lot mixte aragonitique ». On entend par lot mixte aragonitique la forme pulvérulente obtenue après broyage comprenant les deux composants organique et inorganique.
- Le second lot d'une granulométrie de 250 à 50 microns est réservé à l'extraction des biopolymères insolubles et solubles. Il sera dénommé « lot d'extraction des bio-polymères de la couche aragonitique interne ».

La taille et l'éventail des grains des poudres obtenues sont déterminés à l'aide d'un granulomètre laser.

### I.7) Sphérification du lot mixte aragonitique :

Le lot mixte aragonitique est soumis à un traitement mécanique destiné à uniformiser les grains par sphérification, le but étant d'arrondir les angles et les arêtes des grains par frottement.

On place dans un récipient cylindrique en zirconium, à axe de rotation horizontal, comportant des pales en verre d'une largeur variable, un mélange à parts égales de matériau pulvérulent du lot mixte aragonitique et de copeaux de 5mm² de bois dur, par exemple du chêne, stérilisés à l'autoclave.

Les rotations du récipient sont poursuivies pendant une durée et à une vitesse variables, dépendant de la taille du récipient et de la quantité de produit à traiter.

A l'issue du traitement de sphérification, l'ensemble du mélange, lot mixte aragonitique et copeaux, est récupéré dans un récipient inerte rempli d'une quantité adéquate d'eau que l'on agite de manière constante pendant une durée de 15 mn. Après repos de 30 mn, les copeaux de bois flottant en surface sont éliminés par aspiration.

La solution est ensuite filtrée sur un filtre en nylon aux mailles d'un diamètre de 20 microns, puis le culot est séché au Rotavapor^{®} à 40°C et conditionné.

### II. EXTRACTION DES BIO-POLYMERES

### II.1 Extraction des bio-polymères insolubles :

Une quantité adéquate de poudre du lot d'extraction des bio-polymères de la couche aragonitique interne, est mélangée, par aspiration dans la cuve d'admission de la Zone I, avec une quantité suffisante d'eau déminéralisée, pour être injectée en Zone II dans le réacteur à hydrolyse, dans lequel est ajoutée une quantité déterminée d'acide citrique à 25% ; le tout étant refroidi à une température oscillant entre 4 et 5°C sous agitation constante. Le pH, contrôlé par le pH mètre est maintenu au-dessus de 4,5 par adjonction de soude 2,5 N afin d'éviter d'altérer les bio-polymères ; il est ensuite ramené à 7 en fin d'étape par adjonction de 0,1 litre de soude 5N pour 100 litres d'hydrolysat.

Une fois obtenue la dissolution complète de la poudre, l'hydrolysat est transféré dans la cuve de stockage, toujours sous agitation constante, puis transféré dans le séparateur centrifuge où il est soumis à une force de 18 à 20 000G dans le cyclone.

L'opération est répétée si nécessaire après contrôle de la solution par le turbidimètre et correction à l'acide citrique si besoin, la température étant maintenue entre 4 et 5°C.

Selon les résultats fournis par le turbidimètre, l'hydrolysat subit à nouveau une super centrifugation.

A chaque cycle de super centrifugation, le culot des bio-polymères insolubles récolté est lavé et réservé. Les eaux de lavage des culots sont traitées à l'acide oxalique pour vérifier la présence ou non de calcium.

A l'issue de la dernière super centrifugation, on obtient donc un culot contenant tous les bio-polymères insolubles, sous la forme d'un gâteau brunâtre humide, qui est séché par lyophilisation, avec en fin de traitement des sphérules grises de 2 à 3 mm de diamètre, résultant de l'enroulement des protéines sous l'action de la force centrifuge.

Les bio-polymères insolubles extraits sont broyés dans un broyeur planétaire jusqu'à l'obtention d'une poudre d'une granulométrie aléatoire de 5 microns à 100 nanomètres récupérée après tamisage.

### II.2 Extraction des bio-polymères solubles :

Le perméat et les eaux de lavage sont acheminés pour être dessalés dans le dispositif d'assemblage des cassettes d'ultra filtration tangentielle, Millipore^{®} de 1 KD chacune, montées en série pour une surface de 15m², sous une pression de 5 bars avec un débit de 10 à 15 litres par heure, à une température de 40°C.

On ajoute au perméat une quantité suffisante d'acide sulfurique à 2,0 mol/L, afin de provoquer la précipitation des sels de sulfate de calcium.

La solution est filtrée, le perméat est concentré au Rotavapor^{®} sous vide à une température d'ébullition de 33°C afin d'éliminer l'acide citrique sous forme de cristaux.

Le distillat contenant les protéines de faible poids moléculaire ainsi que les ions mono et multivalents, est allongé.

Le seuil de coupure des cassettes ne retenant pas la totalité des protéines et notamment celles de très faible poids moléculaire, le distillat est soumis à une osmose inverse.

Le distillat est alors transféré pour subir un traitement de séparation en phase liquide par perméation à travers des membranes semi-sélectives dont le diamètre des pores de la membrane est de 0,0001 micron, sous l'effet d'un gradient de pression de 40 à 80 bars et.

Le distillat est passé afin de retenir tous les ions mono et multivalents tels que le fer, le magnésium, le zinc, etc ...

Le rétentat récupéré sur les membranes d'osmose inverse est recueilli et allongé avec de l'eau apyrogène, puis concentré par Rotavapor^{®}, sous vide, à une température de 40°C, puis lyophilisé par zéodratation.

On obtient une poudre très fine de couleur blanc grisâtre qui est réservée, puis broyée dans un broyeur planétaire afin d'obtenir après tamisage une poudre d'une granulométrie aléatoire allant de 5 microns à 100 nanomètres.

On vérifie la présence ou l'absence de protéines dans le perméat en prélevant une aliquote de solution qui est traitée par la méthode colorimétrique de Bradford.

### III. CARBONATATION DU CARBONATE DE CALCIUM :

Le carbonate de calcium récupéré après l'extraction des bio-polymères ci-dessus est soumis à un traitement thermique entre 800 et 1100° C, de 20 à 40 mn, puis refroidi à l'air libre de façon lente Ce phénomène est la carbonatation, selon la réaction suivante :

CaCO3 + traitement thermique → Ca(OH)2 + CO2 → CaCO3 + H2O

Lors de cette réaction, le carbonate de calcium se transforme chimiquement en devenant de la chaux, puis sous l'action du CO2 et de l'humidité, redevient du carbonate de calcium amorphe. Cette transformation chimique s'étale sur plusieurs jours en fonction de l'hygrométrie ambiante.

### IV. FORMULATION D'UN MELANGE A PARTIR DU LOT MIXTE ARAGONITIQUE, DES BIO-POLYMERES INSOLUBLES ET SOLUBLES EXTRAITS, ET DU CARBONATE DE CALCIUM TRANSFORME PAR CARBONATATION

Lors de l'extraction des bio-polymères, on a mis en évidence que dans la couche aragonitique interne et la couche calcitique externe des coquilles utilisées, la part des biopolymères insolubles représentait de 2,6% à 4,3% et celle des bio-polymères solubles de 0,4% à 0,7%.

On a préparé le matériau décrit en mélangeant le lot mixte aragonitique, des polymères insolubles obtenus à l'étape II.1, des polymères solubles obtenus à l'étape II.2 et du carbonate de calcium ayant subi une carbonatation obtenu à l'étape III ci-dessus. Les quantités spécifiques des différents composants sont spécifiées dans chacun des exemples d'application donnés ci-après.

Le mélange est effectué dans un mélangeur à couteaux jusqu'à obtention d'une poudre homogène qui est ensuite conditionnée.

### EXEMPLE 2 :

On procède comme dans l'exemple 1, ci-dessus, à l'exception qu'on ajoute une étape de réticulation telle que décrite ci-après à la fin de l'étape I.3.

Dans un récipient translucide en verre ou en plastique, on prépare un mélange d'eau du réseau additionnée de 10% de riboflavine ; l'ensemble est maintenu à une température supérieure à 20°C, l'agitation du mélange générant un flot perpendiculaire aux radiations UVA.

Les coquilles y sont placées verticalement et soumises sur leurs deux faces à l'irradiation de lampes UVA d'une longueur d'ondes de 365 nanomètres seconde, sous une intensité de 2300 micro-joules par centimètre carré pendant 180 mn. L'ensemble étant maintenu sous vide pendant toute la durée du traitement.

Les coquilles sont ensuite rincées et séchées par courant d'air chaud à 40°C.

### EXEMPLE 3 :

Afin de vérifier les propriétés d'adhésivité et de cohésion du carbonate de calcium carbonaté, on procède de la façon suivante:
Dans deux godets Dappen, dénommés respectivement Dappen N°1 et N°2, contenant chacun 1 g de la poudre de nacre obtenue à l'issue de l'étape I.7 du procédé de l'exemple 1, on ajoute :
- 0,1g de carbonate de calcium naturel (Dappen N° 1),
- 0,1g de carbonate de calcium naturel ayant subi une carbonatation, issu de l'étape III du procédé de l'exemple 1 (Dappen N°2).

Après mélange, le contenu de chaque godet Dappen est malaxé avec 2 cc de sang total.

Une photographie de chaque godet Dappen est prise 2 minutes (figure 1) puis 15 minutes (figure 2) après mélange avec le sang total.

Comme illustré sur la Figure 1(1), le mélange du Dappen N°1 reste sous forme d'une poudre rouge, aucun *coagulum* n'est formé. Au bout de 15 minutes, aucun *coagulum* ne s'est formé (Figure 2(1)).

Comme illustré sur la Figure 1 (2), le mélange du Dappen N°2 forme rapidement un *coagulum* et vire progressivement du rouge au brun, prend en masse, peut être modelé, devient collant et durcit au bout de 15 minutes (Figure 2(2)).

### EXEMPLE 4 : Formulation pour substitut osseux extemporané

Un cas de situation clinique critique se présente sous la forme d'une fracture en biseau du canon d'une pouliche de 1 an, traitée par ostéosynthèse. Après l'échec de l'ostéosynthèse traduit par la fracture de 4 vis, une pseudarthrose avec sepsis, suivi d'une fracture secondaire comminutive à petits fragments, laissant comme seule alternative l'euthanasie de l'animal, la décision est prise de l'utilisation du matériau selon l'invention dans la formulation est la suivante :
- 40 g de lot mixte aragonitique d'une granulométrie de 50 nanomètres à 20 microns, issu de l'étape I.8 de l'exemple 1 ;
- 0,070 g de bio-polymères insolubles extraits obtenus à l'étape II.2 de l'exemple 1;
- 0,010 g de bio-polymères solubles extraits obtenus à l'étape II.1 de l'exemple 1;
- 2 g de carbonate de calcium carbonaté issu de l'étape III du procédé de l'exemple 1 ;
- 10 ml de sang veineux autologue pour former un coagulum, modelé sous forme d'un cylindre de 10 cm de longueur sur 2 cm de diamètre, placé dans la perte de substance après ablation des séquestres osseux.

Le membre protégé par des compresses a été plâtré. Des radios post-opératoires ont montré la présence et l'adhésion du substitut osseux selon l'invention, puis une consolidation à 4 mois au terme desquels la pouliche pouvait galoper et sauter des obstacles. Des radios de contrôle pratiquées plus tard ont montré une restitution *ad integrum* du fût osseux avec reconstruction du canal médullaire.

La même formulation a également été utilisée en réalisant un coagulum extemporanément avec 2,5 ml d'eau pour préparation injectable (PPI) à température ambiante.

### EXEMPLE 5 : Formulation d'une crème de cicatrisation cutanée

Une préparation du produit selon l'invention est réalisée d'après la formulation centésimale suivante :
- 10 gr de lot mixte aragonitique d'une granulométrie de 50 nanomètres à 20 microns obtenus selon l'exemple 2 ;
- 0,035 gr de bio-polymères insolubles extraits obtenus à l'étape II.2 de l'exemple 1;
- 0,005 gr de bio-polymères solubles extraits obtenus à l'étape II.1 de l'exemple 1;
- 0,5 gr de carbonate de calcium carbonaté
- 15 gouttes d'un complexe d'huiles essentielles comprenant pour 100 ml :
   Lavandula spica : 1ml
   Salvia officinalis : 2 ml
   Rosa rubiginosa : 10 ml
   Helychrisum italicum : 1,5 ml
   Huile végétale germe de blé : 50 ml
   Huile d'onagre : 10 ml
   Huile d'amande douce : 20 ml
   Emulsion H.E. qsp 100 g

Cette préparation a été appliquée sur une nécrose cutanée du plastron sternal d'un cheval, allant de la base du cou aux ars, sur une hauteur de 32 cm et une largeur de 18 cm. L'observation clinique a montré une cicatrisation exceptionnelle de 1 cm par jour en hauteur et en largeur avec reconstruction des différents plans aponévrotiques, sous-cutané et cutané, et repousse simultanée du poil sans décoloration, avec cicatrisation complète des téguments en 28 jours.

### EXEMPLE 6 : Formulation pour une préparation dermatologique pour le traitement du psoriasis

On sait que le psoriasis est une affection inflammatoire de la peau, caractérisée par le renouvellement accéléré des cellules, sans apoptose, ce qui aboutit à la formation de croûtes épaisses en plaques. En dehors d'une corticothérapie et de traitements locaux à base de goudron et de puvathérapie, dont les résultats sont inconstants et décevants, il existe des traitements plus lourds avec des effets secondaires dangereux pour le patient.

Une préparation centésimale du produit selon l'invention est réalisée selon la formulation suivante :
- 3 gr de bio-polymères insolubles extraits obtenus à l'étape II.2 de l'exemple 1;
- 0,45 gr de bio-polymères solubles extraits obtenus à l'étape II. 1 de l'exemple 1;
- 0,5 gr de carbonate de calcium carbonaté obtenu à l'étape III de l'exemple 1;
- 10 gouttes d'un complexe d'huiles essentielles contenant pour 100 ml :
   Lavandula spica : 1ml
   Salvia officinalis : 2 ml
   Rosa rubiginosa : 10 ml
   Helychrisum italicum : 1,5 ml
   Huile végétale germe de blé : 50 ml
   Huile d'onagre : 10 ml
   Huile d'amande douce : 20 ml
   Emulsion H.E. qsp 100 g

Cette émulsion est appliquée quotidiennement sur les lésions d'un psoriasis sévère au niveau du torse, du dos, des bras et des jambes. Au bout de la troisième application, on constate la disparition des rougeurs, signant la sédation du phénomène inflammatoire, des squames, et la sédation du prurit et des infections surajoutées avec un gain esthétique notoire. L'amélioration des signes cliniques est la traduction des propriétés eutrophiques, anti-phlogistiques et régénératrices des bio-polymères insolubles et solubles.

### EXEMPLE 7 : Formulation d'un pansement cutané pour brûlures

Les propriétés exceptionnelles de régénération des tissus mous des bio-polymères insolubles et solubles extraits selon l'étape II de l'exemple 1, ont été mises en évidence dans un cas de brûlure du second degré profond et du troisième degré après échec de greffe de kératinocytes, avec la formulation suivante :
Pour 100 g :
- 50 g de lot mixte aragonitique d'une granulométrie de 50 nanomètres à 20 microns obtenus selon l'exemple 2 ;
- 0,174 g de bio-polymères insolubles extraits obtenus à l'étape II.2 de l'exemple 1;
- 0,026 g de bio-polymères solubles extraits obtenus à l'étape II.1 de l'exemple 1;
- Cera de Gallien à l'eau de laurier cerise qsp 100 g.

La préparation est appliquée sur la totalité des surfaces brûlées sous pansement occlusif, et renouvelée toutes les 72 heures.

Les examens cliniques répétés ont montré une sédation du phénomène exsudatif, une angiogénèse significative, une sédation de la douleur, une ré-éphitélialisation des zones cruantées et une diminution notable de la mise en tension fibroplastique.

### EXEMPLE 8 : Formulation pour substitut osseux moulé bio-résorbable

Le matériau selon l'invention peut être utilisé pour la fabrication de dispositifs d'ostéosynthèse et d'implants moulés, bio-résorbables.

Selon l'invention, on prépare, pour 100 g :
- 80 gr de lot mixte aragonitique d'une granulométrie de 50 nanomètres à 20 microns obtenu à l'étape I.8 de l'exemple 1 ;
- 0,139 g de bio-polymères insolubles extraits obtenus à l'étape II.2 de l'exemple 1;
- 0,021 g de bio-polymères solubles extraits obtenus à l'étape II.1 de l'exemple 1;
- 20 g de macrogol de gradient 400 ;
- 4 gr de carbonate de calcium carbonaté obtenu à l'étape III de l'exemple 1.

L'ensemble est malaxé dans un mélangeur pendant 10 mn à température ambiante jusqu'à obtention d'une pâte homogène plastique, extrudable et moulable.

On réalise des empreintes de formes adaptées, d'après modélisation numérique de l'anatomie des zones d'insertion éventuelles des dispositifs d'ostéosynthèses et/ou des implants.

Une quantité suffisante de la pâte obtenue précédemment est injectée dans la chambre de compression d'un moule comportant une ou plusieurs empreintes.

L'ensemble est ensuite compressé sous une pression progressive allant de 100 à 220 N; la pression est maintenue pendant un temps variable et décroissant progressivement jusqu'à la valeur 0.

Le dispositif une fois démoulé et séché à 40°C, conditionné sous double emballage, est stérilisé par rayonnement ionisant à 25 KGy.

### EXEMPLE 9 : Préparation pour substitut osseux à biorésorbabilité contrôlée

Il a été constaté que la bio-résorption d'un substitut osseux, ou d'un dispositif bio-résorbable, est directement liée aux diamètres des pores interconnectés, qui doivent varier de 5 à 100 microns, pour permettre sa colonisation par les néo-vaisseaux et les cellules impliquées dans le remodelage osseux.

C'est la raison pour laquelle les inventeurs proposent la réalisation de substituts osseux ou d'implants moulés, dont la porosité interconnectée serait contrôlée.

Pour ce faire on prépare pour 100 g:
- 80 g de lot mixte aragonitique d'une granulométrie de 50 nanomètres à 20 microns obtenus à l'exemple 2 ;
- 0,139 g de bio-polymères insolubles extraits obtenus à l'étape II.2 de l'exemple 1;
- 0,021 g de bio-polymères solubles extraits obtenus à l'étape II.1 de l'exemple 1;
- 20 ml d'une solution d'hydroxypropylméthylcellulose (HPMC) à 50% ;
- 20 mm³ de brins de fils de suture synthétiques mono-filament, résorbables, de 5 mm de long, allant du diamètre 5/0 à 12/0.

Ces fils résorbables sont des polymères tels que l'acide glycolique, le copolymère glycolique, le ε-coprolactone polyglactine (vicryl rapide ou irradié), le chitosan. Ces fils présentent une résorption échelonnée de 12 à 90 jours.

Comme dans l'exemple précédent, la pâte est injectée dans les empreintes d'un moule, puis compressée. Les dispositifs ou implants sont ensuite démoulés, séchés, conditionnés sous double emballage et stérilisés comme précédemment à 25 KGy.

### EXEMPLE 10 : Préparation pour substitut osseux injectable et ciment de scellement d'endoprothèse

On prépare des ciments dont la composition est la suivante pour 100 g:
- 80 g du matériau selon l'invention, composé de :
- 73 gr de lot mixte aragonitique d'une granulométrie de 50 nanomètres à 20 microns obtenu à l'issue de l'étape I.8 ;
- 2,702 g de bio-polymères insolubles extraits obtenus à l'étape II.2 de l'exemple 1;
- 0,405 g de bio-polymères solubles extraits obtenus à l'étape II.1 de l'exemple 1;
- 3,699 g de carbonate de calcium carbonaté issu de l'étape III de l'exemple 1;
- 20 g de HPMC en solution aqueuse haute viscosité à 50%.

Le produit ainsi obtenu est conditionné sous vide ou sous atmosphère contrôlée dans des seringues de contenance variable, de 0,5 cm³ à 1 cm³ par exemple, avec des embouts droits ou coudés conservées au froid à une température d'environ 4° C.

Cette préparation, également utilisable comme ciment de scellement, permet d'éviter, lors du scellement de la queue de prothèse dans la cavité médullaire par exemple, le passage du produit de scellement dans le système circulatoire.

De plus, compte tenu de sa composition, il ne provoque pas de libération de substances volatiles risquant d'impacter le système pulmonaire.

Une telle composition est également proposée pour la vertébroplastie et la cyphoplastie en chirurgie mini-invasive.

### EXEMPLE 11 : Préparation pour substitut osseux à support collagénique

On réalise des substituts osseux ayant la composition suivante :
On prépare pour 100 g :
- 50 g de lot mixte aragonitique d'une granulométrie de 50 nanomètres à 20 microns obtenus à l'étape I.8 de l'exemple 1 ;
- 0,087 gr de bio-polymères insolubles extraits obtenus à l'étape II.2 de l'exemple 1;
- 0,013 gr de bio-polymères solubles extraits obtenus à l'étape II.1 de l'exemple 1;
- 2,5 g de carbonate de calcium carbonaté obtenus à l'étape III de l'exemple 1 ;
- 50 gr de macrogol de gradient 400.

L'ensemble est malaxé jusqu'à obtention d'un gel d'une viscosité de 10 Pa-s environ

On ajoute à ce gel 30 g de Spongia officinalis réduit en fragments d'une taille de 2 mm.

L'ensemble est malaxé jusqu'à obtention d'une pâte homogène d'une viscosité de 10⁸ Pa-s environ. L'ensemble est injecté dans un moule comportant des empreintes de dispositifs d'ostéosynthèse ou d'implants. Après démoulage, les dispositifs ou implants sont séchés, sous courant d'air chaud à 40°C, conditionnés sous double emballage et stérilisés selon le protocole en vigueur.

### EXEMPLE 12 : Préparation pour substitut osseux

Selon un autre mode de réalisation, les bio-polymères extraits de la fraction aragonitique seule et/ou de la fraction calcitique peuvent être ajoutés à tout autre biomatériau d'origine synthétique ou naturelle afin d'optimiser ou d'induire certaines propriétés, notamment des propriétés ostéo-inductrices ou ostéo-mimétiques, dont ils sont dépourvus.

C'est ainsi que des substituts ostéo-conducteurs, tels certains sels de calcium, ont été supplémentés par les bio-polymères extraits de la couche aragonitique, selon la formulation pour 100 g :
- 95 g de granules de βTCP, d'une granulométrie allant de 50 à 250 microns
- 4,4 g de bio-polymères insolubles extraits obtenus à l'étape II.2 de l'exemple 1;
- 0,6 gr de bio-polymères solubles extraits obtenus à l'étape II. 1 de l'exemple 1.

Cette préparation mélangée à du sang autologue est insérée dans un défect osseux créé par l'exérèse d'un kyste à l'apex de l'incisive centrale supérieure.

Dans le même temps, le βTCP seul est compacté dans une perte de substance créée par l'exérèse d'un granulome para apical à la canine supérieure.

Un examen radiologique pratiqué à 2 semaines a montré une densification osseuse plus importante et plus rapide dans la cavité kystique traitée avec le mélange βTCP + bio-polymères insolubles et solubles extraits, que dans la seconde cavité dans laquelle sont bien objectivées les granules de βTCP où seule s'est exprimée l'ostéo-conduction, alors que dans la cavité kystique, l'ostéo-induction est concomitante à l'ostéo-conduction, signant l'acquisition par le βTCP d'une nouvelle propriété.

## Revendications

1. Utilisation des bio-polymères insolubles et solubles extraits de la fraction organique de la couche aragonitique interne et/ou de la couche calcitique externe de la coquille des mollusques bivalves choisis dans le groupe comprenant des Pinctadines, notamment *Pinctada maxima*, *margaritifera*, et des Tridacnes, notamment *Tridacna gigas*, *maxima*, *derasa, tevaroa*, *squamosa*, *crocea, Hippopus hippopus*, *Hippopus porcelanus* comme adjuvants de composition pulvérulente comprenant des sels de calcium, des polymères naturels ou synthétiques, du collagène, des trames minérales de tissus osseux d'origine animale ou humaine,
**caractérisée en ce que** la composition pulvérulente est un matériau semi-synthétique pulvérulent issu d'un biomatériau naturel marin additionné de carbonate de calcium transformé par carbonatation, ledit biomatériau naturel marin étant la couche aragonitique interne de la coquille de mollusques bivalves choisis dans le groupe comprenant des Pinctadines, notamment *Pinctada maxima*, *margaritifera*, et des Tridacnes, notamment *Tridacna gigas*, *maxima, derasa, tevaroa*, *squamosa*, *crocea, Hippopus hippopus*, *Hippopus porcelanus.*

2. Utilisation des bio-polymères insolubles et solubles selon la revendication 1 dans la fabrication de dispositifs, d'implants moulés ou de substitut osseux à formulation extemporanée.

3. Utilisation des bio-polymères insolubles et solubles selon la revendication 1 ou la revendication 2 dans la fabrication de dispositifs ou d'implants moulés à bio-résorption contrôlée comprenant des fils de suture à bio-résorption échelonnée dans le temps.

4. Utilisation des bio-polymères insolubles et solubles selon la revendication 1 pour la formulation de préparations pour substituts osseux à utilisation extemporanée, de substituts osseux à support collagénique poreux, de substituts osseux à trame minérale d'origine animale ou humaine, de dispositifs d'ostéosynthèse et d'implants moulés bio-résorbables, de dispositifs à bio-résorption controlée, de ciments de scellement d'endoprothèses, de ciments injectables pour chirurgie mini-invasive en vertébroplastie et cyphoplastie.

5. Matériau semi-synthétique pulvérulent, issu d'un biomatériau naturel marin, additionné de bio-polymères insolubles et solubles extraits de la fraction organique de la couche aragonitique interne et/ou de la couche calcitique externe de la coquille des mollusques bivalves choisis dans le groupe comprenant des Pinctadines, notamment *Pinctada maxima, margaritifera*, et des Tridacnes, notamment *Tridacna gigas, maxima, derasa, tevaroa*, *squamosa*, *crocea, Hippopus hippopus*, *Hippopus porcelanus* et de carbonate de calcium transformé par carbonatation pour son utilisation pour la cicatrisation et la régénération des pertes de substance ou pour le traitement de brûlures, d'escarres, d'ulcères, de lésions erythémateuses cutanées.

6. Utilisation des bio-polymères insolubles et solubles selon la revendication 1 à 4, ou matériau semi-synthétique pulvérulent pour son utilisation selon la revendication 5, **caractérisé en ce que** le matériau semi-synthétique pulvérulent présente une granulométrie de 5 nm à 100 µm, de préférence de 20 nm à 50 µm, plus préférentiellement encore de 50 nm à 20 µm.

7. Utilisation des bio-polymères insolubles et solubles selon l'une quelconque des revendications 1 à 4 ou la revendication 6, ou matériau semi-synthétique pulvérulent pour son utilisation selon la revendication 6, **caractérisée en ce que** le carbonate de calcium transformé par carbonatation provient d'un carbonate de calcium naturel terrestre, marin ou précipité, ou de la fraction inorganique de la couche aragonitique après extraction des biopolymères insolubles et solubles.

## Patentansprüche

1. Verwendung von unlöslichen und löslichen Biopolymeren, die aus der organischen Fraktion der inneren Aragonit-Schicht und/oder der äußeren Calcit-Schicht der Schale von zweischaligen Mollusken extrahiert sind, die ausgewählt sind aus der Gruppe umfassend Pinctada-Arten, insbesondere *Pinctada maxima, margaritifera,* und Tridacna-Arten, insbesondere *Tridacna gigas, maxima, derasa, tevaroa, squamosa, crocea, Hippopus hippopus, Hippopus porcelanus*, als Zusatzstoffe für eine pulverförmige Zusammensetzung, die Calciumsalze, natürliche oder synthetische Polymere, Kollagen, mineralische Gerüststoffe von Knochengewebe tierischen oder menschlichen Ursprungs umfasst,
**dadurch gekennzeichnet, dass** die pulverförmige Zusammensetzung ein halbsynthetisches pulverförmiges Material ist, das aus einem natürlichen marinen Biomaterial stammt, dem durch Carbonatisierung umgewandeltes Calciumcarbonat zugesetzt wurde, wobei das natürliche marine Biomaterial die innere Aragonit-Schicht der Schale von zweischaligen Mollusken ist, die ausgewählt sind aus der Gruppe umfassend Pinctada-Arten, insbesondere *Pinctada maxima, margaritifera,* und Tridacna-Arten, insbesondere *Tridacna gigas, maxima, derasa, tevaroa, squamosa, crocea, Hippopus hippopus, Hippopus porcelanus.*

2. Verwendung von unlöslichen und löslichen Biopolymeren nach Anspruch 1 bei der Herstellung von Vorrichtungen, geformten Implantaten oder Knochenersatzmaterial mit ex *tempore* Formulierung.

3. Verwendung von unlöslichen und löslichen Biopolymeren nach Anspruch 1 oder Anspruch 2 bei der Herstellung von Vorrichtungen oder geformten Implantaten mit kontrollierter Bioresorption, die Nahtmaterial mit zeitlich gestaffelter Bioresorption umfassen.

4. Verwendung von unlöslichen und löslichen Biopolymeren nach Anspruch 1 zur Formulierung von Zubereitungen für Knochenersatzmaterialien zur Verwendung ex *tempore,* von Knochenersatzmaterialien mit porösem Kollagenträger, von Knochenersatzmaterialien mit mineralischem Gerüst tierischen oder menschlichen Ursprungs, von bioresorbierbaren Osteosynthese-Vorrichtungen und geformten Implantaten, von Vorrichtungen mit kontrollierter Bioresorption, von Zementen zur Versiegelung von Endoprothesen, von injizierbaren Zementen für die minimalinvasive Chirurgie bei der Vertebroplastie und Kyphoplastie.

5. Pulverförmiges halbsynthetisches Material, das aus einem natürlichen marinen Biomaterial stammt und mit unlöslichen und löslichen Biopolymeren versetzt ist, die aus der organischen Fraktion der inneren Aragonit-Schicht und/oder der äußeren Calcit-Schicht der Schalen von zweischaligen Mollusken extrahiert sind, die ausgewählt sind aus der Gruppe umfassend Pinctada-Arten, insbesondere *Pinctada maxima, margaritifera,* und Tridacna-Arten, insbesondere *Tridacna gigas*, *maxima, derasa, tevaroa, squamosa, crocea, Hippopus hippopus, Hippopus porcelanus*, und durch Carbonatisierung umgewandeltes Calciumcarbonat zur Verwendung bei der Heilung und Regeneration von Substanzverlusten oder zur Behandlung von Verbrennungen, Dekubitus, Geschwüren, erythematösen Hautläsionen.

6. Verwendung von unlöslichen und löslichen Biopolymeren nach Anspruch 1 bis 4, oder pulverförmigem halbsynthetischem Material zur Verwendung nach Anspruch 5, **dadurch gekennzeichnet, dass** das pulverförmige halbsynthetische Material eine Korngröße von 5 nm bis 100 µm, vorzugsweise von 20 nm bis 50 µm, noch bevorzugter von 50 nm bis 20 µm aufweist.

7. Verwendung von unlöslichen und löslichen Biopolymeren nach einem der Ansprüche 1 bis 4 oder Anspruch 6 oder pulverförmiges halbsynthetisches Material zur Verwendung nach Anspruch 6, **dadurch gekennzeichnet, dass** das durch Carbonatisierung umgewandelte Calciumcarbonat aus einem natürlichen terrestrischen, marinen oder gefällten Calciumcarbonat oder aus der anorganischen Fraktion der Aragonit-Schicht nach Extraktion der unlöslichen und löslichen Biopolymere stammt.

## Claims

1. Use of insoluble and soluble biopolymers extracted from the organic fraction of the aragonitic inner layer and/or of the calcitic outer layer of the shell of bivalve molluscs selected from the group comprising Pinctadines, notably *Pinctada maxima, margaritifera,* and Tridacnes, notably *Tridacna gigas, maxima, derasa, tevaroa*, *squamosa*, *crocea, Hippopus hippopus, Hippopus porcelanus*, as additives of pulverulent composition comprising calcium salts, natural or synthetic polymers, collagen, mineral structures of bony tissues of human or animal origin,
**characterized in that** the pulverulent composition is a pulverulent semisynthetic material derived from a natural marine biomaterial with addition of calcium carbonate transformed by carbonation, said natural marine biomaterial being the aragonitic inner layer of the shell of bivalve molluscs selected from the group consisting of Pinctadines, notably *Pinctada maxima, margaritifera*, and Tridacnes, notably *Tridacna gigas, maxima, derasa, tevaroa, squamosa*, *crocea, Hippopus hippopus*, *Hippopus porcelanus.*

2. Use of insoluble and soluble biopolymers according to Claim 1 in the manufacture of devices, moulded implants or bone substitutes formulated for extemporaneous use.

3. Use of insoluble and soluble biopolymers according to Claim 1 or Claim 2 in the manufacture of devices or moulded implants with controlled bioabsorption, comprising suture threads with bioabsorption staggered over time.

4. Use of insoluble and soluble biopolymers according to Claim 1 for the formulation of preparations for bone substitutes for extemporaneous use, for bone substitutes with a porous collagen support, for bone substitutes with a mineral framework of human or animal origin, for bioabsorbable osteosynthesis devices and moulded implants, for devices with controlled bioabsorption, for cements for sealing endoprostheses, and for injectable cements for minimally invasive surgery in vertebroplasty and kyphoplasty.

5. Pulverulent semisynthetic material, obtained from a natural marine biomaterial, with addition of insoluble and soluble biopolymers extracted from the organic fraction of the aragonitic inner layer and/or of the calcitic outer layer of the shell of bivalve molluscs selected from the group consisting of Pinctadines, notably *Pinctada maxima, margaritifera,* and Tridacnes, notably *Tridacna gigas, maxima, derasa, tevaroa, squamosa*, *crocea, Hippopus Hippopus*, *Hippopus porcelanus*, and calcium carbonate converted by carbonation, for its use for the healing and regeneration of losses of substance or for the treatment of burns, sores, ulcers and erythematous skin lesions.

6. Use of insoluble and soluble biopolymers according to Claims 1 to 4, or pulverulent semisynthetic material for its use according to Claim 5, **characterized in that** the pulverulent semisynthetic material has a granulometry of 5 nm to 100 µm, preferably of 20 nm to 50 µm, more preferably of 50 nm to 20 µm.

7. Use of insoluble and soluble biopolymers according to any one of Claims 1 to 4 or Claim 6, or pulverulent semisynthetic material for its use according to Claim 6, **characterized in that** the calcium carbonate transformed by carbonation derives from a natural terrestrial, natural marine or precipitated calcium carbonate, or from the inorganic fraction of the aragonitic layer after extraction of the insoluble and soluble biopolymers.
